# EUROPÄISCHE PATENTSCHRIFT

(11) **EP 0 684 244 B1**
(45) Veröffentlichungstag und Bekanntmachung des Hinweises auf die Patenterteilung: **10.10.2001**
(21) Anmeldenummer: 95107419.4
(22) Anmeldetag: 16.05.1995
(51) Int. Cl.: C07D 513/16, A61K 31/50

(54) **1,9-überbrückte Thiazolo(3,2-a)chinolinderivate**
1,9-bridged thiazolo(3,2-a)quinoline derivatives
Dérivés de thiazolo(3,2-a)quinoline pontés en positions 1,9

(30) Priorität: 27.05.1994 DE 4418510
(43) Veröffentlichungstag der Anmeldung: 29.11.1995
(73) Patentinhaber: BAYER AG, 51368 Leverkusen (DE)
(72) Erfinder: Jaetsch, Thomas, Dr., D-50668 Köln (DE); Hallenbach, Werner, Dr., D-40789 Monheim (DE); Himmler, Thomas, Dr., D-51519 Odenthal (DE); Mielke, Burkhard, Dr., D-51375 Leverkusen (DE); Bremm, Klaus Dieter, Dr., D-45661 Recklinghausen (DE); Endermann, Rainer, Dr., D-42113 Wuppertal (DE); Pirro, Franz, Dr., D-40764 Langenfeld (DE); Stegemann, Michael, Dr., D-51381 Leverkusen (DE); Wetzstein, Heinz-Georg, Dr., D-51377 Leverkusen (DE)

(56) Entgegenhaltungen:
- EP-A- 0 286 089
- EP-A- 0 387 877
- EP-A- 0 472 826
- EP-A- 0 520 240
- EP-A- 0 523 512
- EP-A- 0 550 903
- EP-A- 0 563 734
- EP-A- 0 588 166
- JOURNAL OF MEDICINAL CHEMISTRY, Bd. 36, Nr. 18, 1993 Seiten 2621-2626, Y. JINBO ET AL.
- EUROPEAN JOURNAL OF MEDICINAL CHEMISTRY, Bd. 26, Nr. 9, Dezember 1991 Seiten 889-906, M. OGATA ET AL.

## Beschreibung

Die vorliegende Erfindung betrifft 1,9-überbrückte Thiazolo[3,2-a]chinolinderivate, Verfahren zu ihrer Herstellung sowie diese enthaltende antibakterielle Mittel.

Es ist bereits bekannt geworden, daß Thiazolochinolincarbonsäuren antibakteriell wirksam sind. Beispiele hierfür finden sich in EP-A 286 089, EP-A 387 877, EP-A 472 826, EP-A 588 166 und im Journal of Medicinal Chemistry 36, 2621 (1993). Desweiteren sind aus EP-A- 563 734 verwandte Chinolincarbonsäuren und ihre Verwendung zur Herstellung von Arzneimitteln bekannt.

Es wurden nun Verbindungen der allgemeinen Formel (I) gefunden 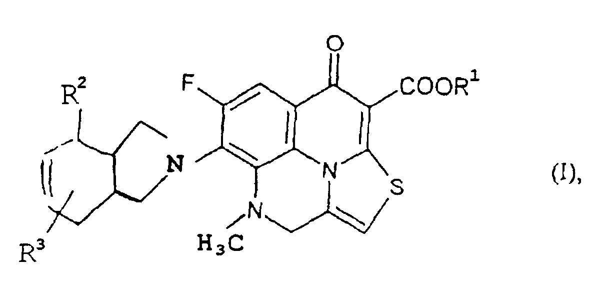 in welcher
- R¹: für Wasserstoff, gegebenenfalls durch Hydroxy, Methoxy, Amino, Methylamino oder Dimethylamino sustituiertes Alkyl mit 1 bis 4 Kohlenstoffatomen oder (5-Methyl-2-oxo-1,3-dioxol-4-yl)-methyl steht,
- R²: für -NR⁴R⁵, -CH₂-NR⁴R⁵, steht,
wobei
R⁴ für Wasserstoff, Alkoxycarbonyl mit 1 bis 4 C-Atomen im Alkoxyteil oder C₁-C₃-Acyl steht,
R⁵ für Wasserstoff oder Methyl steht,
- R³: für Wasserstoff, geradkettiges oder verzweigtes C₁-C₃-Alkyl oder Cyclopropyl steht,

Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- oder Guanidiniumsalze vorliegen.

Man erhält die Verbindungen der Formel (I), wenn man Verbindungen der Formel (II) 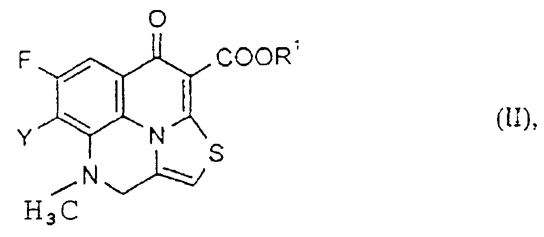 in welcher
- R¹: die oben angegebene Bedeutung hat und
- Y: für Fluor oder Chlor steht,
mit Verbindungen der Formel (III)
in welcher 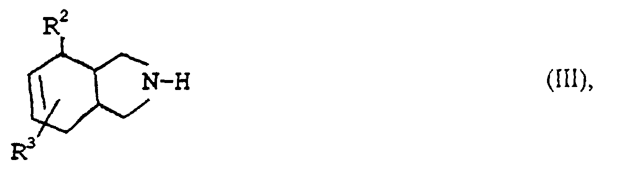
- R² und R³: die oben angegebenen Bedeutungen haben
gegebenenfalls in Gegenwart von Säurefängern umsetzt.

Die erfindungsgemäßen Verbindungen weisen im Vergleich zu bekannten Vertretern dieses Strukturtyps eine höhere antibakterielle Wirkung insbesondere im grampositiven Bereich auf. Sie eignen sich daher als Wirkstoffe für die Human- und Veterinärmedizin.

Im folgenden seien folgende Verbindungen der Formel (I) genannt:

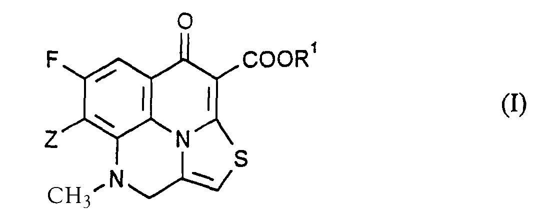

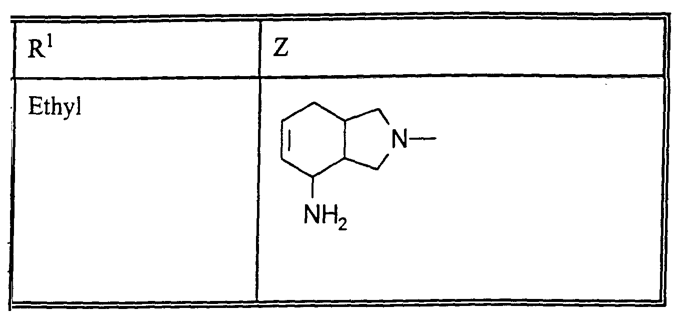

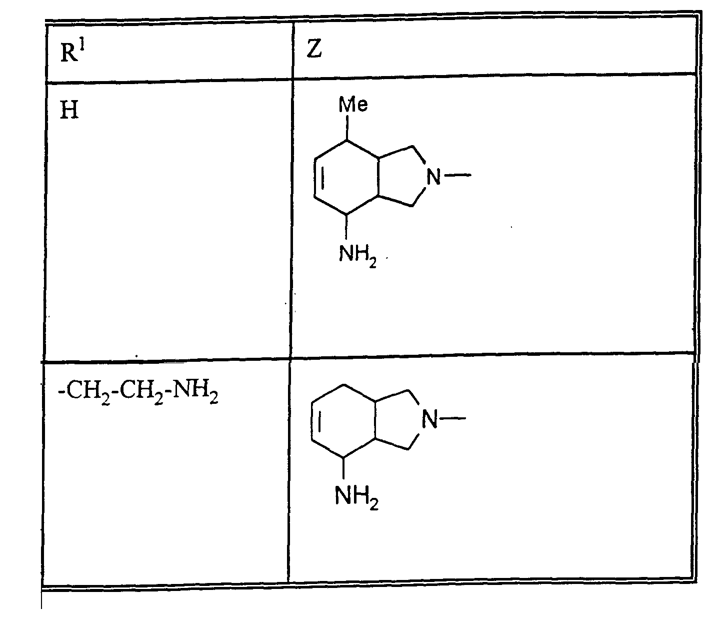

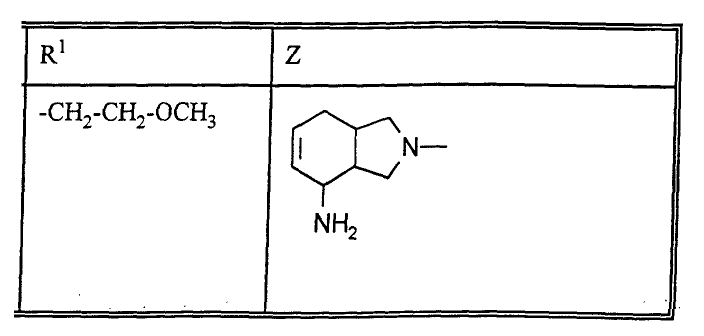

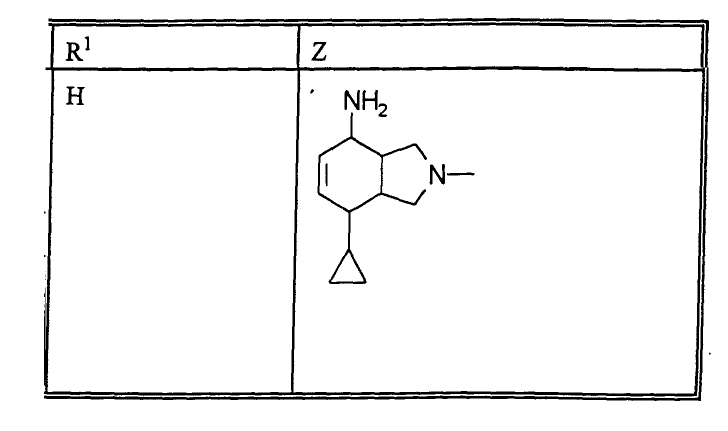

Die als Ausgangsverbindungen verwendeten Verbindungen der Formel (II) sind bekannt, bzw. können nach bekannten Verfahren hergestellt werden. Sie können gegebenenfalls als Racemate, Enantiomere oder reine Diastereomere eingesetzt werden.

Als Beispiele seien genannt:
7,8-Difluor-5-oxo-9,1-[(N-methylimino)methano]-5H-thiazolo[3,2-a]chinolin-4-carbonsäure
7,8-Difluor-5-oxo-9,1-[(N-methylimino)methano]-5H-thiazolo[3,2-a]chinolin-4-carbonsäureethylester

Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als Beispiele seien genannt:
2-Amino-8-azabicyclo[4.3.0]non-3-en 2-Methylamino-8-azabicyclo[4.3.0]non-3-en 4-Methyl-2-methylamino-8-azabicyclo[4.3.0]non-3-en 5-Methyl-2-methylamino-8-azabicyclo[4.3.0]non-3-en 2-Methylaminomethyl-8-azabicyclo[4.3.0]non-3-en 5-Isopropyl-2-methylamino-8-azabicyclo[4.3.0]non-3-en 2-Amino-5-isopropyl-8-azabicyclo[4.3.0]non-3-en 2-Amino-5-methyl-8-azabicyclo[4.3.0]non-3-en 2-Amino-5-cyclopropyl-8-azabicyclo[4.3.0]non-3-en

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, . N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäue, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen unterhalb von Konzentrationen ähnlicher Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Micrococcus luteus und Enterococcus faecalis beobachtet werden.

Auch gegenüber Bakterien, die gegenüber vergleichbaren Substanzen als weniger empfindlich eingestuft werden, insbesondere resistenten Staphylococcus aureus und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektions- und orale verabreichbare Lösungen, Suspensionen und Emulsionen, ferner Pasten, Salben, Gele, Cremes, Lotions, Puder und Sprays genannt.

Die Wirkstoffe eignen sich bei günstiger Warmblütertoxizität bevorzugt zur Bekämpfung von bakteriellen Erkrannkungen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese, unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose; Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die minimalen Hemmkonzentrationen (MHK) der erfindungsgemäßen Verbindungen wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen aufgeführt.

**Tabelle**

| MHK-Werte | | | | |
|---|---|---|---|---|
| Spezies | Stamm | Beispiel Nr. | | |
| | | 2 | 4 | 5 |
| Staphylococcus aureus | ATCC 29123 | ≦0,015 | ≦0,015 | ≦0,015 |
| | 133 | ≦0,015 | ≦0,015 | ≦0,015 |
| | ICB 25701 | 0,125 | 0,125 | 0,25 |
| | ICB 25768 | 0,25 | 0,5 | 1 |
| Enterococcus faecalis | 27 101 | 0,125 | 0,062 | 0,125 |
| | 9790 | 0,125 | 0,062 | 0,125 |
| Micrococcus luteus | 9341 | 0,125 | 0,062 | 0,25 |

### Herstellung der Wirkstoffe

### Beispiel 1 (Referenzbeispiel für das Herstellungsverfahren, keine erfindungsgemäße Verbindung)

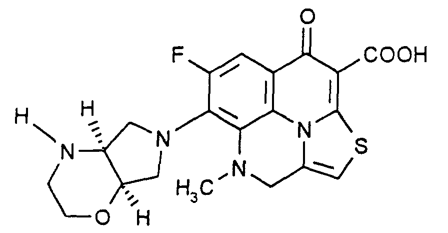

### 7-Fluor-8-(1R,6S)-2-oxa-5,8-diazabicyclo[4,3,0]nonan-8-yl)-5-oxo-9,1-(N-methylimino)methanol-5H-thiazolo[3,2-a]chinolin-4-carbonsäure

150 mg (0,465 mmol) 7,8-Difluor-5-oxo-9,1-[(N-methylimino)methano]-5H-thiazolo[3,2-a]-chinolin-4-carbonsäure werden mit 119 mg (0,93 mmol) (IR,6S)-2-Oxa-5,8-diazabicyclo-[4,3,0]nonan in 3 ml Dimethylsulfoxid drei Stunden unter Argon auf 120 bis 130°C erwärmt. Die Mischung wird im Hochvakuum eingeengt, der Rückstand aus Ethanol umkristallisiert und getrocknet.
Ausbeute: 149 mg (74 % der Theorie)
Schmelzpunkt: >300°C

### Beispiel 2

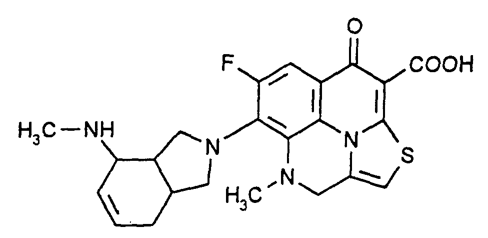

### 7-Fluor-8-(2-methylamino-8-azabicyclo[4,3,0]non-3-en-8-yl)-5-oxo-9,1-[(N-methylimino)-methano]-5H-thiazolo[3,2-a]chinolin-4-carbonsäure

Analog zum Beispiel 1 wird bei der Umsetzung mit 2-Methylamino-8-azabicyclo[4,3,0]non-3-en die Titelverbindung erhalten. Schmelzpunkt: 228°C (unter Zersetzung)

### Beispiel 3

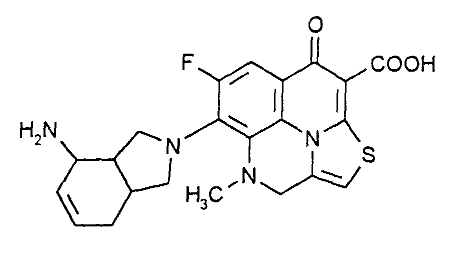

### 8-(2-Amino-8-azabicyclo[4,3,0]non-3-en-8-yl)-7-fluor-5-oxo-9,1-[(N-methylimino)-methano]-5H-thiazolo[3,2-a]chinolin-4-carbonsäure

Analog zum Beispiel 1 wird beider Umsetzung mit 2-Amino-8-azabicyclo[4,3,0]non-3-en die Titelverbindung erhalten.
Schmelzpunkt: >300°C

### Beispiel 4

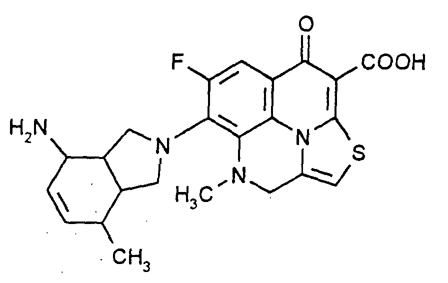

### 8-(2-Amino-5-methyl-8-azabicyclo[4,3,0]non-3-en-8-vl)-7-fluor-5-oxo-9,1-(N-methylimino)methanol-5H-thiazolo[3,2-a]chinolin-4-carbonsäure

Analog zum Beispiel 1 wird bei der Umsetzung mit 2-Amino-5-methyl-8-azabicyclo[4,3,0]non-3-en die Titelverbindung erhalten.
Schmelzpunkt: >300°C

### Beispiel 5

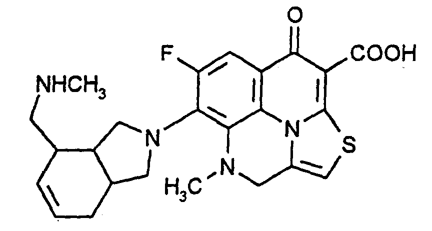

### 7-Fluor-8-(2-methylaminomethyl-8-azabicyclo[4,3,0]non-3-en-8-yl)-5-oxo-9,1-[(N-methylimino)methano]-5H-thiazolo[3,2-a]chinolin-4-carbonsäure

Analog zum Beispiel 1 wird bei der Umsetzung mit 2-Methylaminomethyl-8-azabicyclo[4,3,0]non-3-en die Titelverbindung erhalten.
Schmelzpunkt: 210 bis 220°C (unter Zersetzung)

## Patentansprüche

1. Verbindungen der allgemeinen Formel (I) 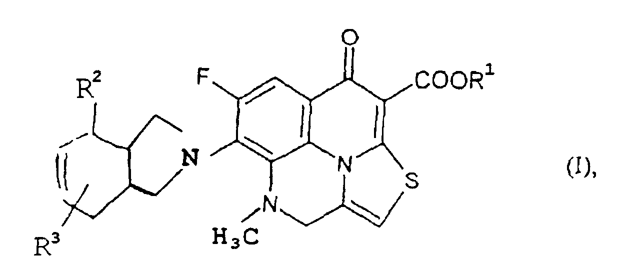 in welcher
R¹ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
R² für -NR⁴R⁵, -CH₂-NR⁴R⁵,steht,
wobei
R⁴ für Wasserstoff, Alkoxycarbonyl mit 1 bis 4 C-Atomen im Alkoxyteil oder C₁-C₃-Acyl steht,
R⁵ für Wasserstoff oder Methyl steht,
R³ für Wasserstoff, geradkettiges oder verzweigtes C₁-C₃-Alkyl oder Cyclopropyl steht,
in Form ihrer Racemate oder enantiomerenreinen Verbindungen sowie in Form ihrer pharmazeutisch verwendbaren Hydrate und Säureadditionssalze sowie in Form ihrer Alkali-, Erdalkali-, Silber- oder Guanidiniumsalze.

2. Verfahren zur Herstellung der Verbindungen der Formel (I) gemäß Anspruch 1
**dadurch gekennzeichnet, daß** man Verbindungen der Formel (II) 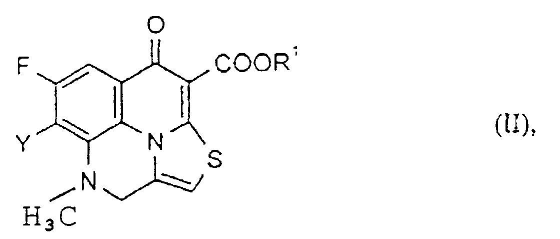 in welcher
R¹ die in Anspruch 1 angegebene Bedeutung hat und
Y für Fluor oder Chlor steht,
mit Verbindungen der Formel (III) 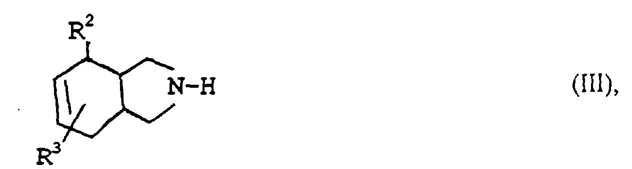 in welcher
R² und R³ die in Anspruch 1 angegebene Bedeutung haben,
gegebenenfalls in Gegenwart von Säurefängern umsetzt.

3. Arzneimittel enthaltend Verbindungen der Formel (I) gemäß Anspruch 1.

4. Verwendung von Verbindungen der Formel (I) gemäß Anspruch 1 zur Herstellung von Arzneimitteln.

5. Verwendung von Verbindungen der Formel (I) gemäß Anspruch 1 bei der Herstellung antibakterieller Mittel.

## Claims

1. Compounds of the general formula (I) 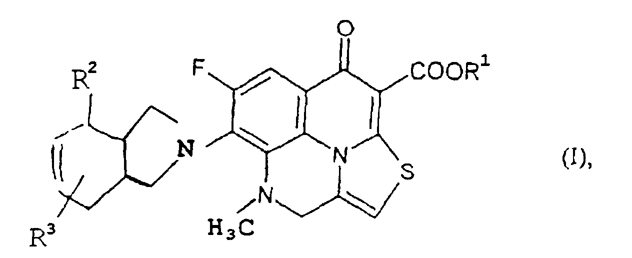 in which
R¹ represents hydrogen, optionally hydroxyl-, methoxy-, amino-, methylamino- or dimethylamino- sustituted alkyl having 1 to 4 carbon atoms or (5-methyl-2-oxo-1,3-dioxol-4-yl)-methyl,
R² represents -NR⁴ R⁵, -CH₂-NR⁴R⁵,
where
R⁴ represents hydrogen, alkoxycarbonyl having 1 to 4 carbon atoms in the alkoxy moiety or C₁-C₃-acyl ,
R⁵ represents hydrogen or methyl,
R³ represents hydrogen, straight-chain or branched C₁-C₃-alkyl or cyclopropyl,
in the form of their racemates or enantiomerically pure compounds and in the form of their pharmaceutically utilizable hydrates and acid addition salts and in the form of their alkali metal, alkaline earth metal, silver or guanidinium salts.

2. Process for the preparation of the compounds of the formula (I) according to Claim 1,
**characterized in that** compounds of the formula (II) 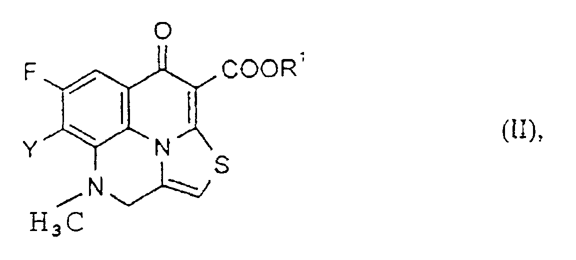 in which
R1 has the meaning given in Claim 1 and
Y represents fluorine or chlorine,
are reacted with compounds of the formula (III) 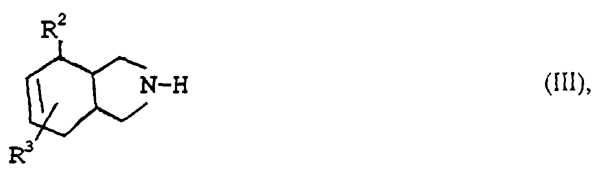 in which
R² and R³ have the meaning given in Claim 1,
if desired in the presence of acid scavengers.

3. Medicaments comprising compounds of the formula (I) according to Claim 1.

4. Use of compounds of the formula (I) according to Claim 1 for the preparation of medicaments.

5. Use of compounds of the formula (I) according to Claim 1 in the preparation of antibacterial compositions.

## Revendications

1. Composés de formule générale (I) 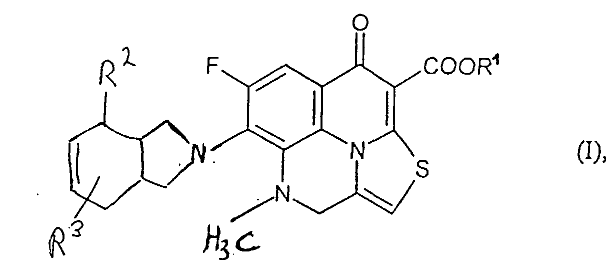 dans laquelle
R¹ représente l'hydrogène, un groupe alkyle ayant 1 à 4 atomes de carbone éventuellement substitué par un radical hydroxy, méthoxy, amino, méthylamino ou diméthylamino, ou un groupe (5-méthyl-2-oxo-1,3-dioxole-4-yl)-méthyle,
R² représente un groupe -NR⁴R⁵, -CH₂-NR⁴R⁵,
où
R⁴ est l'hydrogène, un groupe alkoxycarbonyle ayant 1 à 4 atomes de carbone dans la partie alkoxy ou un groupe acyle en C₁ à C₃,
R⁵ est l'hydrogène ou un groupe méthyle,
R³ est l'hydrogène, un groupe alkyle en C₁ à C₃ linéaire ou ramifié ou un groupe cyclopropyle,
sous forme de leurs racémates ou de composés énantiomères purs ainsi que sous forme de leurs hydrates et de leurs sels d'addition d'acides acceptables du point de vue pharmaceutique de même que sous forme de leurs sels de métaux alcalins, de métaux alcalino-terreux, d'argent ou de guanidinium.

2. Procédé de production des composés de formule (I) suivant la revendication 1,
**caractérisé en ce qu'**on fait réagir des composés de formule (II) 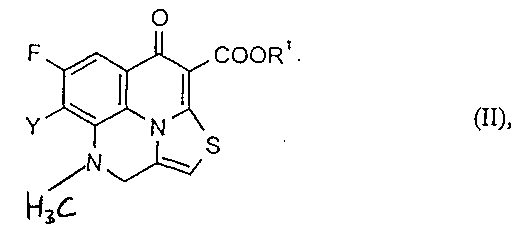 dans laquelle
R¹ a la définition indiquée dans la revendication 1 et
Y représente le fluor ou le chlore,
avec des composés de formule (III) 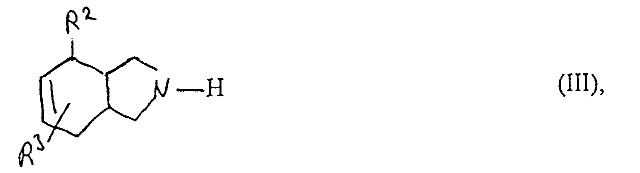 dans laquelle
R² et R³ ont la définition indiquée dans la revendication 1, le cas échéant en présence d'accepteurs d'acides.

3. Médicaments contenant des composés de formule (I) suivant la revendication 1.

4. Utilisation de composés de formule (I) suivant la revendication 1 pour la préparation de médicaments.

5. Utilisation de composés de formule (I) suivant la revendication 1 dans la préparation d'agents antibactériens.
